Europäisches Patentamt

European Patent Office

Office européen des brevets

(11) Publication number: **0 391 444**

**A2**

(12) **EUROPEAN PATENT APPLICATION**

(21) Application number: **90106698.5**

(22) Date of filing: **06.04.90**

(51) Int. Cl.⁵: **A61K 37/02, A61K 9/14, A61K 9/08, A61K 47/26, A61K 47/10**

(30) Priority: **07.04.89 US 335175**

(43) Date of publication of application:
**10.10.90 Bulletin 90/41**

(84) Designated Contracting States:
**AT BE CH DE DK ES FR GB GR IT LI LU NL SE**

(71) Applicant: **SYNTEX (U.S.A.) INC.**
**3401 Hillview Avenue**
**Palo Alto California 94303(US)**

(72) Inventor: **Tsai, Kelly Peng**
**752 Talisman Court**
**Palo Alto, CA 94303(US)**
Inventor: **Visor, Gary Conard**
**560 Talbot Avenue**
**Pacifica, CA 94044(US)**
Inventor: **Knepp, Victoria Marie**
**1259 16th Avenue**
**San Francisco, CA 94122(US)**
Inventor: **Gu, Leo Cheeliang**
**12134 Beauchamps Lane**
**Saratoga, CA 95070(US)**
Inventor: **Chiang, Hi-Shi**
**1524 Pam Lane**
**San Jose, CA 95120(US)**

(74) Representative: **Barz, Peter, Dr. et al**
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) **Interleukin-1 Formulation.**

㊗ A lyophilized dosage form for human recombinant interleukin-1 (IL-1) is disclosed. Specifically described is a lyophilized dosage form of interleukin-1$\beta$ which retains its intrinsic potency for a minimum of one year when stored at ambient temperature.

## Interleukin-1 Formulation

This invention relates to an injectable dosage form of Interleukin-1 (IL-1). The formulation is produced as a sterile lyophilized product which when reconstituted is suitable for administration in humans.

IL-1 is a protein hormone that has been described to exist in two forms, IL-1$\alpha$ and IL-1$\beta$. Coding sequences for both proteins have been described and produced by recombinant technology. The expression system employs genetically engineered E.coli containing DNA coding for the human protein. Details of the expression, fermentation and purification of these proteins have been well documented. (See, for example, March, et.al., Nature 315:641 (1985); Kronheim, et.al., Biotechnology 4:1078 (1986))

IL-1 mediates a wide range of intercellular responses that directly affect the hematopoietic status and immune functions of a host organism. (Oppenheim, et.al., Immunol. Today 3:113 (1982); Rosenwasser, et.al., J. Exp. Med. 150:709 (1979); Mizel, Immunol. Rev. 63:51 (1982)) IL-1 is manufactured and released by actuated macrophages and monocytes that are mobilized in response to infection or injury. The physiological responses induced by IL-1 include neutrophilia, lymphocyte activation, inflammation and the systemic "acute phase response" (Gauldie, et.al., Immunology 60:203 (1987)).

The formulation of recombinant proteins is a significant problem which requires consideration of numerous factors which can affect both the chemical and physical stability and thus the activity of the protein over time. A recent review of the many factors which must be considered when attempting to stabilize proteins in a pharmaceutically acceptable formulation is described by Wang, Y.J. and M.A. Hansen, J. Parenter.Sci. Technol. 42(Suppl. 2S) Technical Report No. 10 (1988). Use of excipients and other stabilizers are discussed with emphasis on both the advantages and inherent problems associated with each.

U.S. Patent 4,604,377 describes the formulation of the recombinant protein IL-2 in a stable, lyophilized form. The formulation comprises a water soluble carrier, such as mannitol, combined with a detergent such as sodium dodecyl sulfate (SDS) in order to retain the solubility of the protein in water.

European Patent Application, EP 0 229 016 describes the formulation of a stable IL-2 composition for use in treating humans. The formulation may be freeze-dried in the presence of human serum albumin (HSA) at physiological pH. The described formulation additionally requires the inclusion of sugar or a sugar alcohol, for example, mannitol in order to retain its biological activity.

U.S. Patent 4,645,830 describes a stabilized form of IL-2 in which HSA is used in the formulation. Data is presented which shows the formulated protein can be freeze-dried and stored for up to one month.

While all members of the family of cytokines, including, for example, IL-1, IL-2, IL-6 and CSFs, are recognized as having an effect on the immune system, each individual protein has its own biophysical characteristics along with different mode of action. For this reason, a formulation for one of the cytokines, such as IL-2 or CSF, would not necessarily translate into a workable formulation for IL-1. For example IL-2 differs significantly from IL-1 in terms of water solubility and potency.

This invention provides for a stable, highly active pharmaceutical composition of IL-1 suitable for parenteral administration in humans. The formulation comprising a therapeutically effective amount of IL-1 has been shown to retain its biological activity for more than one year at ambient temperature without formation of degradation products or concomitant loss of activity.

In one embodiment of the invention, a pharmaceutical composition suitable for parenteral administration is described which comprises an amorphous, lyophilized mixture of a biologically active recombinant IL-1 protein and a pharmaceutically acceptable excipient, preferably in matrix form.

In another embodiment of the invention an aqueous pharmaceutical composition suitable for administration in humans, comprising IL-1, polyhydroxylated, non-protein IL-1 compatible compounds and a pharmaceutically acceptable liquid for injection is described.

In another embodiment of the invention, a formulation of biologically active IL-1$\beta$ suitable for injection into humans is described. In its preferred form, the formulation consists of 100 $\mu$g (0.01%) of essentially pure IL-1$\beta$ combined with 3% by weight mannitol and 3% by weight sucrose in 0.12% tromethamine buffer, pH 7.5.

In yet another embodiment of the invention, a process for the production of a biologically active protein suitable for injection into humans is described. The process comprises lyophilizing an aqueous solution of essentially pure recombinant IL-1 protein with IL-1 compatible compounds in a tromethamine buffer.

Brief Description of Figure

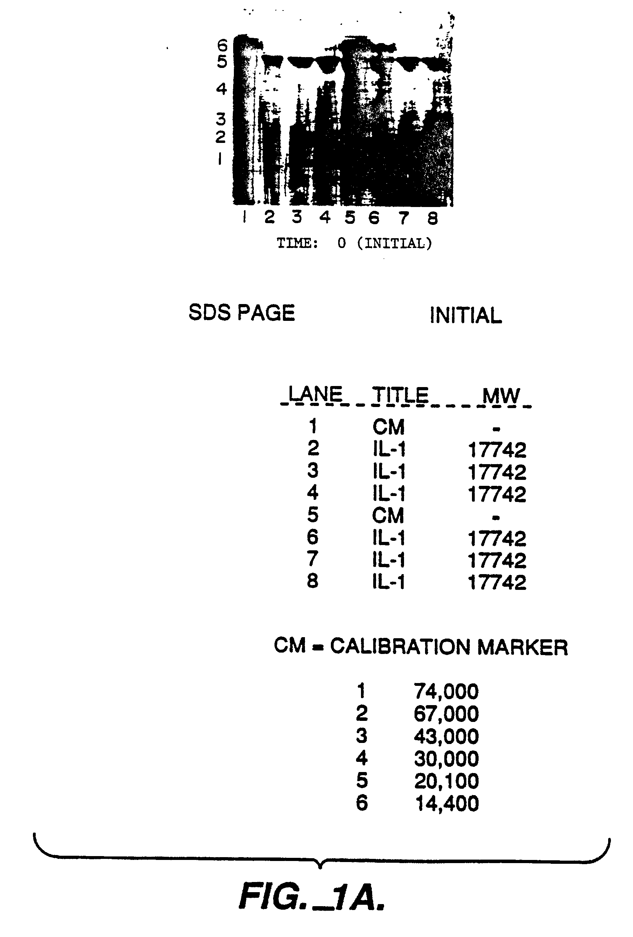

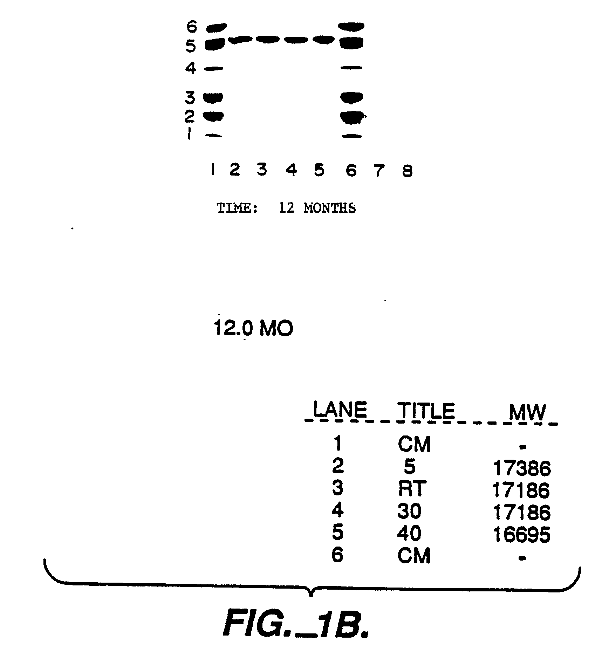

Figure 1: SDS-Polyacrylamide gel electrophoresis (SDS-PAGE) showing stability of product over time

For purposes of this invention, biologically active recombinant interleukin-1 protein (IL-1) includes both IL-1α and IL-1β and thus the composition of this invention can be used for both the alpha and beta form of IL-1. IL-1β is characterized by a primary sequence of 153 amino acid residues with two cysteine residues that do not form a disulfide linkage. The protein has an isoelectric point (pI) of 6.9 (Meyers, et.al., J. Biol. Chem. 262(23):11176 (1987) and a molecular weight of 17,377. IL-1α is characterized by a primary sequence of 159 amino acids with one cysteine residue. By isoelectric focusing, the protein has a major and a minor species with corresponding pIs of 5.45 and 5.2. (Wingfield, et.al. Eur. J. Biochem. 165:537 (1987). The molecular weight of IL-1α is 18,100.

As used in this invention, the following definitions shall apply:

"Parenterally administratable" means capable of administration to the body by means of an injection. Injections can be administered by various routes, e.g. into or through the skin, through the mucous and the serous membranes, lymphatic administration or intrathecal administration, to name a few, and includes of cause infusion administration (and use of infusion pumps).

"Amorphous" as used in pharmaceutical applications means not in crystalline form. Crystallization is a phenomenom characteristic of the solutes present in the aqueous solution during freeze drying. Certain solutes, for example, salts, will crystallize unless present in very low amounts. When in very low amounts the salts will exist in a mixed crystalline-amorphous state. In contrast, excipients, such as, sucrose, polyvinylpyrrilidone (PVP) and the like, will not crystallize from aqueous solutions during freeze drying. Such solutes are thus retained in the amorphous form upon freeze drying. Whether or not a composition is amorphous can be determined in standard manner, for example by X-ray diffraction.

"Lyophilization" means the freeze drying process whereby an aqueous solution has the water removed so as to form a stable solid preparation. The process involves freezing then dehydration of the sample under high vacuum.

A suitable "Excipient" according to the invention includes polyhydroxylated, non-protein IL-1 compatible compounds capable of maintaining the biologically active IL-1 in an amorphous, stable form over an extended period of time; and preferably forming a matrix.

"IL-1 compatible compounds" shall mean one or more pharmaceutical compounds which when added to the IL-1 protein environment will not adversely interact with the IL-1 so as to reduce its biological activity or otherwise change the desirable characteristics of the protein. Examples of IL-1 compatible compounds include mannitol, sucrose, inositol and sorbitol.

"Physiologically acceptable pH" means the numerical value of the hydrogen ion concentration present in the body, e.g., blood has a physiological pH of 7.4.

"Polyhydroxylates" refer to polyhydric alcohols having several hydroxyl groups located at various carbon positions on the molecule, e.g. sugars, glycerol. It is believed these compounds act to stabilize protein conformations in solution. In a water-polyhydroxylate system, a protein can be prevented from unfolding by the preferential exclusion of the polyhydroxylate from the protein domain. This phenomenom produces a more favorable environment in which there is closer contact between the solvent, i.e. water, and the protein. The preferential interactions of proteins with water result in a shell of hydration forming around the protein. (Gekko and Timasheff, Biochemistry 20:4667 (1981)).

The composition of this invention is designed for parenteral administration. Parenteral administration is generally characterized by injection, either subcutaneously, intramuscularly or intravenously.

In the preferred practice of the invention, the IL-1 is formulated as a lyophilized product for parenteral administration.

The development of a stable parenteral dosage form for a recombinant protein requires the evaluation of a multitude of factors. Included among some of the more significant factors to be considered are: 1) solution stability at sub-ambient, ambient, and elevated temperatures; 2) route of administration, e.g. intravenous, bolus, infusion, intramuscular; 3) frequency of administration; 4) dose range; 5) adsorptive interactions and compatability with processing delivery devices and fluids; 6) selection and qualification of container closure systems; 7) sterile processing parameters, e.g. bioburden, membrane surface area, batch size, filtration pressure, in-process controls, and the like; 8) validation requirements; 9) quality control; and 10) release specifications. (See also, Evans, et.al. J. Parenter. Sci. Technol. 40:83 (1986))

Initial efforts were focused on the development of a lyophilized, intravenous dosage form of IL-1β and IL-1α. Details of the low temperature transitions and sublimation processes associated with freeze-drying proteins have been well documented. (See, Hellman, et.al., Biochim. Biophys.Acta 749:133 (1983); Carpenter, et.al., Biochim.Biophys.Acta 923:109 (1987); Margolis and Eisen, Lancet 12:1345 (1984)). In addition, there are a number of considerations that must be addressed when preparing a protein solution to undergo the freeze-drying process. Some of the important parameters to be considered are listed in Table 1.

Table 1
Process Related Parameters Associated with the Lyophilization of Recombinant Proteins
Excipient Selection:
Bulking Agents (e.g. mannitol, inositol, lactose)
Buffers (pH of maximum stability, pI, influence on adsorption)
Tonicity Agents (e.g. mannitol, lactose, sorbitol)
Cryoprotectants (e.g. polyhydric alcohols, human serum albumin (HSA), bovine serum albumin (BSA), amino acids, heavy metals, mannitol, glycerol, PVP, sucrose, methyl cellulose, cyclodextrins)
Special additives (e.g. preservatives, antioxidants, chelating agents)
Control Variables During Cyclic Development And Validation:
Freezing rate (i.e. ultimate temperature)
Vacuum control and measurement
Shelf temperature /heating rate
Primary, secondary and terminal drying phases and characterization
Residual moisture analysis, unloading specifications
Stoppering Control (e.g. vacuum, atmospheric pressure, argon and nitrogen addition)
Manufacturing Variables
Bioburden
Adsorption
Foaming or shearing stress during filtration and filling
Membrane qualification and sterility assurance levels
In process controls, finished product specifications
Fill volume and container size
Scale-up

Each of the parameters listed in Table 1 were considered in the development of the formulation described by this invention. The formulation which was found to provide the most stable composition in a workable excipient matrix was a matrix of mannitol and sucrose in equal amounts buffered using tromethamine. It was found that the ratio of mannitol to sucrose in the composition directly affected the physical integrity, i.e. the ability of the composition to form a stable and integral preparation prior to lyophilization thereby ensuring success in the lyophilization.

In a typical lyophilization cycle of the invention, samples of IL-1 solutions in a concentration of from 1 to 100 µg/ml, preferably 10 to 100 µg/ml, such as 20 µg/ml and 100 µg/ml, are vialed in glass vials and placed within a precooled lyophilization chamber. The lyophilization cycle proceeds in a controlled manner through the various regions, i.e. pre-freezing, primary drying, and secondary drying until a final lyophilized product is obtained. During the course of the lyophilization cycle, the temperature is maintained at approximately -40°C during the first 5 hours. As the freeze drying process proceeds, the temperature gradually increases over approximately a 15 to 30 hour time period until it is again at room temperature (approximately 22°C). The cycle is maintained at room temperature until the desired water content is achieved, i.e. measurements taken at time intervals indicate that for the IL-1 protein the water content is from 0.5 to 5%, preferably from 0.5 to 2%. The pressure throughout the lyophilization cycle is approximately 100 microns of Hg. The time, temperature and pressure are dependent on product load and heat transfer characteristics, therefore, the exact paramaters will vary to some degree for each individual run.

The final lyophilized product is designed by its formulation to have the following characteristics: 1) cryoprotection of the protein during freezing and drying cycles; 2) sufficient strength to withstand long term storage; 3) uniform consistency; 4) sufficient dryness; 5) rapid reconstitution; and 6) pyrogen and particulate free upon reconstitution.

Since the composition must be administered in a form compatible with and non-toxic to the recipient, the choice of excipient in the formulation of the product is very important. Not only must the composition be compatible to the recipient, it must also provide an environment in which the recombinant protein will retain its stability and potency. For that reason, while surfactants such as SDS or polysorbate 80 are frequently employed in protein formulations, it is preferable to avoid such detergents due to their potential interference in metabolism of the drug, as well as interference in the monitoring assays. Likewise, the use of additional proteins, such as HSA or globulins, are suitably only employed when needed with very low active ingredient levels.

In general, excipient systems used with recombinant proteins are composed of non-protein bulking agents, buffers, tonicity agents, cryoprotectants and in some instances, preservatives, antioxidants or chelating agents. All of these excipients must be of compendial grade, i.e., a quality that will not

4

compromise the pharmaceutical acceptance of the protein. The precise components which are necessary as excipients for a recombinant protein are dependent on the characteristics of the particular protein and the form in which the pharmaceutical composition is to be formulated.

In particular, mannitol is a well-characterized bulking excipient that provides mechanical support to the formulation matrix thereby allowing it to maintain its shape without collapsing during the freeze drying process.

Sucrose is used in the formulation primarily as an adjunct bulking agent, tonicifier and stabilizer. Polyhydroxy compounds, such as sucrose, provide protein stability at both low and high temperatures based on their ability to impart a shell of hydration to the macromolecules. (See, Gekko and Timasheff, supra). As water is intimately associated with the native state of all proteins both at the surface and within internal cavities, the presence of hydrogen donor/acceptor molecules can be very important during the sublimation process.

In the preferred composition of the invention, mannitol and sucrose are both used.

Another factor in the determination of the preferred formulation is the selection of the buffer system. The buffer system used is especially important in the formulation of the protein. Factors considered when selecting a buffer system include the determination of the preferred pH for maximum stability of the particular protein, the pI, and the effect on adsorption.

While any suitable buffer can be used, such as citrate buffer, in the preferred practice of the invention, the polyhydric compound, tromethamine (Tris(hydroxymethyl)aminomethane), is the buffer system of choice. This tertiary amine has a pKa of 7.9, and therefore, its ability to control the target pH of 7.5 is very good even at low temperatures. In addition, since the protein has a pI of 6.9, the selected pH will help to maintain a net negative balance on the molecule, thereby helping to minimize interactions with the negatively charged walls of glass containers. Also, since the buffer itself is a polyhydric compound, its ability to participate in a hydration network surrounding the protein is a distinct advantage (Chaillot, et.al. Am. J. Hematology 10:319 (1981)).

Of importance in any freeze-dried system, whether for a protein or small molecule, are the ratios of the various excipients. These ratios can dramatically influence both the freezing and drying processes as well as the overall molecular state, i.e. amorphous as opposed to crystalline, of the material surrounding the protein. In the preferred practice of the invention it was determined that optimal results could be obtained when the ratio of mannitol to sucrose was equal.

The significance of this aspect is related to the ability of amorphous constituents to stabilize or buffer the protein, whereas components that crystallize out of solution during lyophilization are unable to do so. Relative amounts of additives therefore have the ability to either facilitate or inhibit the eutectic crystallization of one another, and thus determine the ultimate effectiveness of the preparation.

In a preferred practice of the invention, the excipient material selected for use with the recombinant IL-1 consists of a ternary solute system composed of mannitol, sucrose, and tromethamine buffer adjusted to a pH of 7.5.

Preferably, the concentration of sucrose and mannitol is from 1 to 5% by weight, most preferrably 3% by weight. The tromethamine is preferrably used in a concentration range of 0.01% to 0.5%. More preferrably, in a concentration range of 0.05% to 0.18%. Most preferrably, the concentration of tromethamine used is 0.12% which corresponds to a molar concentration of 0.01.

With the lower levels of active ingredient, such as 20 μg IL-1, suitably 0.1 to 2%, more suitably 0.5 to 1% HSA, such as 1% HSA, are also included in the composition.

The composition of this invention is prepared by (a) mixing the IL-1 with an aqueous solution of the excipients at an appropiate pH; (b) removing any contaminants in the solution by sterile filtration to give a sterile solution; and (c) lyophilizing the resulting sterile solution under non-destructive conditions so as to retain the amorphous nature of the composition.

In step (a), the aqueous solution of the excipients is prepared for example by dissolving compendial grade mannitol, sucrose and tromethamine in sterile water for injection to give the desired concentration. This maybe done at any appropriate temperature, e.g. 5°C to 100°C, but generally is done at ambient temperature for convenience. Once the excipients are dissolved, the pH is adjusted to the desired range, that is, a pH which is non-destructive to the protein and physiologically acceptable to a patient. The pH range is about 6.5 to 8.0, preferrably about 7.2 to 7.8, most preferably about 7.5. The pH is adjusted by using appropriate amounts of solutions of an acid and base. A suitable acid is 1N HCl and a suitable base is 1N NaOH.

In particular, the selection of an equivalent ratio of sucrose to mannitol in the lyophilized formulation of IL-1 helps to maintain an amorphous matrix. The matrix acts to protect the protein from potentially denaturing freeze-concentration effects. In order to verify the amorphous property of the lyophilized environment surrounding the IL-1 protein, photomicrographs and X-ray powder diffraction scans were taken.

The X-ray powder diffraction studies of the lyophilized IL-1 showed characteristics of an overall amorphous morphology. Examination of the lyophilized IL-1 under the microscope further confirmed the noncrystalline properties of the excipients around the active protein.

Stability evaluation of the lyophilized product demonstrated that the formulated system has sufficient stability and bioactivity for extended clinical studies. These studies utilized both an in vitro biological assay, e.g., thymocyte proliferation assay, as well as a number of biophysical techniques to establish the viability of the product. A more detailed account of the assays used can be found in the Examples. For illustration here, data shown in Example 3, Table 4 indicate that a preferred sterile, lyophilized IL-1β composition according to Example 1 can be stored at either 5°C or room temperature for a minimum of two years. This is exceptionally high and advantageous stability.

The stability studies were carried out over a 12 month time period at temperatures of 5°C, room temperature (RT) and 40°C. The long term studies were carried out in order to eliminate the problems frequently associated with attempting to interpret accelerated storage stability data. Difficulty in interpreting accelerated stability data often results from the fact that accelerated storage conditions do not necessarily extrapolate or confirm stability data generated under actual long term storage conditions.

Various biophysical techniques can be used to monitor the protein. Such techniques include, for example, reverse-phase high performance liquid chromatography (RP-HPLC), enzyme-linked immunosorbent assays (ELISA), SDS-polyacrylamide gel electrophoresis (SDS-PAGE), isoelectric focusing (IEF), and circular dichroism (CD) analysis. Results from these biophysical assays are then correlated with biological activity assessments provided by an in vitro cell culture assay, such as the mouse thymocyte proliferation assay.

Results from the various biophysical assays performed indicate fairly consistent values for protein content even though the assay techniques are based on vastly different physiochemical properties. For example, the assay results for IL-1β via RP-HPLC correlate well with the immunochemical results obtained from ELISA.

The analysis of proteins by SDS-PAGE in reduced and non-reduced gel systems provides a sensitive means via silver staining of analyzing any covalent aggregation caused by degradation due to thermal instability. The lyophilized product of formulated IL-1β analyzed in this manner failed to reveal the formation of products other than the parent monomeric form (approx. 17,377 daltons).

IEF did provide a degradation profile consistent with the observed loss of biological activity at high temperatures. Comparison of IEF profiles run on the initial IL-1 material with the same material after 6 months and 12 months of storage at 5°C, room temperature and 40°C indicated degradation over time when the IL-1 was stored at 40°C. The multiple bands observed at 40°C suggest the presence of deamidated species which are significantly lower in charge distribution or possess lower pIs than the normal IL-1β banding pattern.

Evaluation of biological activity on samples stored under a variety of conditions failed to show significant changes until almost nine months into the study. At that point it became evident activity was dramatically reduced in samples stored at 40°C. Formulated product stored at 5°C and room temperature for the same period remained viable and maintained potency relative to reference standard material.

CD analyses were carried out on stock and lyophilized samples of both IL-1α and IL-1β to determine whether lyophilization changes the conformation of these proteins. Comparison of the spectra of the stock samples and their lyophilized analogues showed only minor differences, indicating that lyophilization does not affect the conformational (tertiary) structure of the proteins.

The amount of active protein administered to a human subject will be dependent upon the subject being treated, the severity of the affliction, the manner of administration and the judgement of the prescribing physician. In each instance, a therapeutically effective amount based on the indication being treated will be administered.

A therapeutically effective amount of IL-1 means an amount of IL-1α or IL-1β that is sufficient to mediate the intercellular responses that affect the hematopoietic status or immune functions of a host organism. Thus the amount present in the composition will be sufficient to create an immunologic response when administered to a subject in need of such treatment. For example, a person with a suppressed immune system due to chemotherapy, radiotherapy or disease could be treated using this formulation.

Generally, the amount of active protein administered to elicit a therapeutic response will be about 0.001 to 5.0, for example 0.002 to 5.0 μg/kg/day, preferably about 0.01 to 0.1 μg/kg/day, such as .05 μg/kg/day. Clinical studies conducted for specific indications will determine the ideal dosage for the individual treatment regimes.

For administration in humans, the lyophilized pharmaceutical composition is reconstituted in a phar-

6

maceutically acceptable solution for injection. Such solutions include, for example, sterile water for injection, sterile saline for injection or dextrose in a sterile aqueous formulation. The amount of solution used will be dependent on the desired final concentration of biologically active protein for administration. In the preferred practice of the invention, the lyophilized pharmaceutical composition is reconstituted in 1.0 ml of sterile water for injection to yield a total dosage amount per vial of 1-100 μg of IL-1 protein. This dosage amount can then be diluted by the physician administering the composition to the appropriate concentration using an appropriate sterile isotonic admixture, e.g., saline for injection (0.9%) or dextrose for injection (5% by weight). Conveniently the composition can be presented in two vials, one containing the lyophilized powder, one containing the liquid for reconstitution.

Examples

The examples which follow are illustrative, and not limiting of the invention.

Example 1: Preparation of IL-1α and IL-1β Sterile Lyophilized Product

This example sets forth a representative composition of the invention and a method for its preparation.

Compendial grade excipient materials were used in the manufacture of the drug product. Table 2 presents the formulation for IL-1β. The formulation of IL-1α was carried out with identical conditions and ingredients.

Table 2

| Formulation of IL-1β | | |
|---|---|---|
| Compound/Excipient | Concentration | Percent |
| | (per ml) | (%w) |
| IL-1β Protein | 0.0001 gram | 0.0100 |
| Mannitol (USP) | 0.0300 gram | 3.0000 |
| Sucrose (NF) | 0.0300 gram | 3.0000 |
| Tromethamine (USP) | 0.0012 gram | 0.1200 |
| Hydrochloric acid (NF) | to adjust pH 7.5 +/- 0.2 | |
| Sodium Hydroxide (NF) | to adjust pH 7.5 +/- 0.2 | |
| Water for injection | qs 1.0000 ml | |
| Nitrogen (NF) | | |

Compounding of the formulation involved sequential dispersement of mannitol (3% w), sucrose (3% w) and tromethamine, in sterile water for injection. The compounding was carried out in a glass lined compounding vessel. Following solubilization of all additives, the pH of the solution was measured and adjusted with 1N HCl or 1N NaOH to a pH of 7.5

A stock solution of IL-1β having a protein content of 1.56 mg/ml was added to the compounding vessel with low speed mixing using a magnetic stirrer. The final concentration of protein in solution was 100 μg/ml as determined by in-process HPLC and spectrophotometric analyses.

The bulk solution was then sterile-filtered through a PVDF (polyvinylidene difluoride) membrane (Millipore Corp., Bedford, MA.) with a pore size of 0.22 μm and run at pressures of less than 5 psi. Sterile-filtered product was then filled into 5 ml Type I glass vials at 1 ml per vial and partially covered with lyophilization stoppers for freeze-drying.

All vialed products were equilibrated at 5°C for 30 minutes within the chamber before the lyophilization process was initiated.

Upon completion of the lyophilization process, the chamber was returned to atmospheric pressure with sterile, dry nitrogen. Vialed material was stoppered under sterile conditions and sealed with aluminum caps.

[The lyophilization process was carried out as described in the "Description of the Invention", achieving a 2% water content. This example has been carried out on various scales, ranging from 30 ml to a 5 litre

7

batch. The data given in the following Examples 2-6 was generated on product from the 5 litre batch.]

Example 2: ELISA determination of IL-1β concentration

This example sets forth a method to determine the concentration of IL-1β in buffer solution. A modification of the ELISA method developed by Kenney, et.al. J. Immunology 138:4236 (1987) was used.

Monoclonal antibody (100 μl per well of a 10-15 μg/ml solution) was coated onto vinyl assay plates by incubation overnight at 5°C. The wells were washed with phosphate buffered saline (PBS) containing 0.05% thimerosal and 0.1% bovine serum albumin (BSA) and countercoated with 200 μl of PBS containing 5% non-fat dry milk and 0.05% thimerosal for one hour at room temperature. After washing the wells, 50 μl/well of sample or standard and 50 μl of biotinylated IL-1β monoclonal antibody in PBS containing 1% non-fat dry milk and 0.05% thimerosal was added and the plates were incubated for two hours at room temperature. After washing each well, 100 μl/well of an optimized solution of horseradish peroxidase conjugated strepavidin was added and the plates were incubated for one hour. The wells were then washed. 100 μl of ortho-phenylenediamine (OPD) substrate solution (1 mg/ml OPD:0.3% $H_2O_2$:0.1 M citrate buffer, pH 4.5) was added and the plates were incubated in the dark for 30 minutes. The absorbance at 450 nm was determined for each well and used to calculate the concentration of IL-1β reference to a standard curve.

The quantification limit of the assay is 30 pg/ml. The usable range of the standard curve is from 30 to 2000 pg/ml. The interassay and intra-assay variability for samples in buffer is 11.6% and 3.12%, respectively.

Table 3

| Determination of IL-1β concentration(μg/ml) by ELISA in Example I composition. | | | |
|---|---|---|---|
| At the times indicated, samples of stored powder were dissolved in water and the IL-1β concentration determined. | | | |
| Time (months) | Storage Temperature | | |
| | 5°C | RT | 40°C |
| 0 | * | 96.0± 5.4 | * |
| 1.0 | 94.7± 6.3 | * | 88.0±4.2 |
| 1.5 | * | 112.3± 8.3 | * |
| 3.0 | 93.4± 1.6 | * | 84.1±4.3 |
| 3.5 | * | 93.5± 3.5 | * |
| 6.0 | 92.4± 4.3 | * | 79.3±5.3 |
| 6.5 | * | 95.3± 6.3 | * |
| 9.0 | 100.0± 1.8 | * | 77.3±5.7 |
| 9.5 | * | 100.0± 3.0 | * |
| 12.0 | * | * | 58.2±12 |
| 12.5 | 92.0± 3.1 | * | * |
| 13.0 | * | 88.5± 4.9 | * |
| 18.0 | 74.0± 4.6 | * | * |
| 18.5 | * | 75.7± 4.9 | * |
| 24.0 | 99.8±10.2 | * | * |
| 24.5 | * | 102.1±10.1 | * |
| * = not determined | | | |

Example 3: Determination of IL-1 activity by means of a Thymocyte Proliferation Bioassay

This example sets forth the method used to determine the biological activity of the IL-1 formulation. A thymocyte proliferation bioassay designed to measure the biological activity of IL-1$\alpha$ and IL-1$\beta$ was carried out using a modification of the assay method as first described by Gery, et.al., J. Exp. Med. 136:128 (1972).

Diluted IL-1$\alpha$ and IL-1$\beta$ standards or samples and phytohemagglutinin were added to cultures of C3H/HeJ mouse thymocytes 1x10$^6$/well) in modified minimum essential medium with 5% fetal bovine serum, 25mM HEPES buffer, 50 U/ml penicillin, 50 $\mu$g/ml streptomycin, 2mM L-glutamine, 20 $\mu$g/ml gentamicin and 5 x 10$^{-5}$ M 2-mercaptoethanol. After 48 hours at 37°C, 0.5 $\mu$Ci/well of [$^3$H]-thymidine was added and the cultures were incubated overnight. The cells were collected onto glass fiber filters using an automatic cell harvester and the radioactivity incorporated into the cells was determined by scintillation counting. From a curve fitted to the log of the data points for the [$^3$H]-thymidine uptake versus either the concentration or dilution factor of the standards, the concentration or dilution factor of the standard which produces 50% (ED-50) of the maximum uptake was estimated.

The ED-50 of the reference standard (pg/well) is by definition equal to one unit of activity. The ED-50 of the standard divided by the ED-50 of the sample is equal to the percent of activity present in the sample. The intra- and interassay variability of this method is 17.3 and 26.0%, respectively. As presented in Table 4, the activity in Units/$\mu$g is calculated by dividing the ED-50 of the standard by the ED-50 of the sample and multiplying by the Units/$\mu$g in the standard.

Table 4

| Bioactivity of IL-1$\beta$ in Units/$\mu$g x 10$^5$, In Example 1 Composition | | | |
|---|---|---|---|
| Time (months) | Temperature | | |
| | 5°C | RT | 40°C |
| 0 | * | 1.56±.259 | * |
| 1.0 | 1.50±.324 | 1.56±.218 | 1.25±.121 |
| 3.0 | 1.78±.351 | * | 1.51±.081 |
| 3.5 | * | 1.74±.376 | * |
| 6.0 | 2.14±.294 | * | 1.51±.157 |
| 6.5 | * | 1.89±.189 | * |
| 9.0 | 1.5.±.197 | * | 0.83±.104 |
| 9.5 | * | 1.26±.262 | * |
| 12.0 | 1.41±.32 | 1.07±.110 | 0.73±.232 |
| 18.0 | 1.17±.431 | 0.89±.134 | * |
| * = not determined | | | |
| Bioactivity of IL-1$\alpha$ in Units/$\mu$g x 10$^5$ | | | |
| Time (months) | Temperature | | |
| | 5°C | RT | 40°C |
| 0 | 1.38±.14 | n/d | n/d |
| 2.0 | 1.87±.15 | n/d | n/d |
| 3.5 | 1.83±.16 | n/d | n/d |
| 6.0 | 1.74±.16 | n/d | n/d |
| 9.0 | 1.29±.12 | n/d | n/d |
| n/d = not determined | | | |

Example 4: Identification and quantitation of IL-1$\beta$ using reverse-phase HPLC

This example sets forth a method for determining the amount of undegraded IL-1 protein present after lyophilization.

The lyophilized powder of IL-1β, Example 1, was reconstituted with 1 ml of sterile water for injection and gently mixed until the solid was completely dissolved. An aliquot of the reconstituted solution was injected into a high pressure liquid chromatography system, Hewlett-Packard 1090 HPLC (Hewlett-Packard, Palo Alto, Ca.) and separated on a reverse-phase HPLC column (SynchropakR RP-8 or equivalent) by gradient elution.

Typical chromatographic operating conditions were as follows:

| Detection wavelength: | 220nm |
|---|---|
| Injection volume: | 10μl |
| Flow rate: | 0.5ml/min |
| Temperature: | ambient |
| Detector range: | 0.05 AUFS (absorbance units full scale) |
| Pressure: | 1000 psi |
| Amount injected | 1.0 μg |
| Mobile phase: | (A) water |
| | (B) 90:10 acetonitrile:water |
| (both A and B contain 0.1% trifluoroacetic acid) | |
| Gradient: | 35% (B) at 0.0 min |
| | 2% (B)/min for 18 min |
| | 2.5% (B)/min for 4 min |
| | 12.5% (B)/min for 4 min |

IL-β identity is confirmed by comparison of its retention time in the sample chromatogram to its retention time in the calibration standard chromatogram.

Additionally, the identity of IL-1β can be confirmed by comparison of the spectrum of the peak in the sample chromatogram with the spectrum of the IL-1β peak in the calibration standard chromatogram.

Table 5

| Stability of IL-1$\beta$ as determined by RP-HPLC (% LS) | | | |
|---|---|---|---|
| Time (months) | Temperature | | |
| | 5°C | RT | 40°C |
| 0 | * | 100.5±3.21 | * |
| 1.0 | 102.0±1.41 | * | 98.5±0.71 |
| 1.5 | * | 101.1±1.41 | * |
| 2.5 | 98.0±0 | * | * |
| 3.0 | * | * | 92.5±0.71 |
| 3.5 | 98.0±0 | 98.5±0.71 | * |
| 6.0 | 99.5±0.71 | * | 79.0±0 |
| 6.5 | * | 96.5±0.71 | * |
| 9.0 | 98.5±0.71 | * | 80.5±0.71 |
| 9.5 | * | 97.5±0.71 | * |
| 12.0 | 97.5±0.71 | * | 70.5±0.71 |
| 12.5 | * | 96.0±2.83 | * |
| 18.0 | * | * | * |
| 18.5 | 102.3±0.96 | * | * |
| 19.0 | * | 97.5±1.0 | * |
| 25.5 | 99.0±0.0 | * | * |
| %LS = Labelled strength (100µg/vial = 100%) * = not determined | | | |

## Example 5: Identification and determination of purity using Isoelectic Focusing (IEF)

This example sets forth yet another method for determining the purity of the lyophilized IL-1 protein.

IL-1$\beta$ lyophilized powder of Example 1 was reconstituted with 1 ml of sterile water for injection. The sample solution and calibration markers of known pIs were applied to PhastGels (Pharmacia Inc., Piscataway, N.J.) and isoelectric focusing was carried out using the Pharmacia PhastSystem instrument following the instructions provided by the manufacturer.

The gels were silver stained. Optimum sample concentration for silver stained gels was found to be 100ng/µl for IL-1$\beta$.

The isoelectric points of IL-1$\beta$ were determined by interpolating from the migration distances of the isoelectric point calibration markers. Comparison of IEF profiles run on the initial IL-1 material with the same material after 6 months and 12 months of storage at 5°C, room temperature and 40°C indicated degradation over time when stored at 40°C. After 6 months at 40°C representative IEF profile showed 4 major bands with at least 10 extraneous bands. After 12 months the 4 major bands were visible, however, the number of extraneous bands had increased significantly. Over the same time period, the IEF profiles for material stored at 5°C or room temperature remained essentially unchanged. The multiple bands observed at 40°C suggest the presence of deamidated species which are significantly lower in charge distribution or possess lower pIs than the normal IL-1$\beta$ banding pattern.

## Example 6: Identification and determination of purity and molecular weight by SDS-PAGE

This example sets forth a confirmatory assay for determination of the purity of the lyophilized product. In addition, determination of the molecular weight by SDS-Page confirms the absence of degradation products over time.

IL-1$\beta$ lyophilized powder of Example 1 was reconstituted with 1 ml of sterile water for injection and diluted with buffer containing sodium dodecyl sulfate (SDS). After the protein was denatured, the sample solutions and calibration markers of known molecular weights were applied to PhastGels (Pharmacia, Inc., Piscataway, N.J.). SDS-PAGE was carried out using the Pharmacia PhastSystem instrument according to the procedures recommended by the manufacturer. The gels were silver-stained and the migration distances of the bands measured.

The molecular weight of IL-1$\beta$ was determined by interpolating from the migration distances of the molecular weight calibration markers. Figure 1 shows representative SDS-PAGE results for the formulated producted stored over a 12 month time period.

Example 7. A Further IL-1$\beta$ Composition.

Example 1 was repeated, but with the following changes:

(i) 1%(w) HSA was additionally incorporated. The HSA was added, with the slowest possible mixing, just prior to the addition of the IL-1$\beta$; and

(ii) 20 $\mu$g(per ml) of IL-1$\beta$ was used.

Example 8. Further Stability Data

The stability of the Example 7 composition was also examined. Table 6 below shows the data currently available.

Table 6

| Stability of IL-1$\beta$ as determined by RP-HPLC (% L.S.) - initial [IL-1$\beta$] = 20 $\mu$g/ml | |
| --- | --- |
| Storage Condition | |
| Time (mos) | 5$^{\circ}$C |
| 0.0 | 98.7 ± 1.0 |
| 1.0 | 109 ± 0.8 |
| 1.5 | * |
| 2.0 | 108.5 ± 0.8 |
| 3.0 | 100.8 ± 2.2 |

Example 9

The lyophilized powders of Examples 1 and 7 were reconstituted with 1 ml of sterile water for injection, in sufficient weight to give respectively 100 $\mu$g and 20 $\mu$g of IL-1$\beta$ per ml. of liquid.

**Claims**

1. A pharmaceutical composition suitable for forming a parenterally administratable aqueous formulation, which composition comprises an amorphous, lyophilized mixture of a biologically active recombinant interleukin-1 protein (IL-1) and a pharmaceutically acceptable excipient.

2. The composition according to claim 1, wherein the excipient forms a matrix.

3. The composition of claim 2 wherein the excipient matrix is present in an amount sufficient to maintain the mixture in amorphous form over an extended period of time and establish a physiologically acceptable pH when reconstituted in a pharmaceutically acceptable solution for injection.

4. The composition of claim 1, 2 or 3 wherein the excipient matrix is a combination of (a) polyhydroxylated, non-protein, IL-1 compatible compounds with (b) tromethamine.

5. The composition of any of the preceding claims wherein the biologically active interleukin-1 is IL-1 beta (IL-1$\beta$).

6. The composition of any one of claims 1-4 wherein the biologically active interleukin-1 is IL-1 alpha (IL-1$\alpha$).

7. The composition of any one of the preceding claims wherein the IL-1 compatible compounds are mannitol and sucrose in equal concentrations of from 1% to 5% by weight (% w).

8. The composition of claim 7 wherein the IL-1 compatible compounds are mannitol and sucrose in equal concentrations of 3% by weight.

9. A composition according to any one of the preceding claims, comprising 0.010% IL-1$\beta$.

10. A composition according to any one of the claims 1-8, comprising 0.002% IL-1$\beta$.

11. A composition according to claim 10, additionally comprising HSA.

12. A composition according to any one of the preceding claims, in aqueous solution form.

13. A composition according to claim 12, which composition comprises IL-1 polyhydroxylated, non-protein, IL-1 compatible compounds and a pharmaceutically acceptable liquid for injection buffered to a physiologically acceptable pH.

14. The aqueous composition of claim 12 or 13 which is buffered using tromethamine at a concentration range of 0.01 % w to 0.5 % w.

15. The composition of claim 12, 13 or 14 wherein the IL-1 is IL-1$\beta$ at a concentration in the range of 1 to 100 $\mu$g/ml.

16. A composition according to claim 12 of biologically active IL-1 suitable for injection into humans consisting essentially of:
a) 1-100 $\mu$g/ml of essentially pure IL-1;
b) 1-5% weight volume (w/v) mannitol;
c) 1-5% w/v sucrose; and
d) 0.01-5% tromethamine.

17. The composition of claim 16 in which the biologically active IL-1 is IL-1$\beta$.

18. A composition according to claim 17, of biologically active IL-1$\beta$ suitable for injection into humans comprising:
a) 0.01% of essentially pure IL-1$\beta$;
b) 3% weight volume (w/v) mannitol;
c) 3% w/v sucrose; and
d) 0.12% tromethamine.

19. A composition according to claims 16 or 17, wherein the IL-1 or IL-1$\beta$ concentration is 0.002%, and which additionally comprises 1% HSA.

20. A process for the production of a composition according to any one of the preceding claims, wherein the process comprises lyophilizing an aqueous solution of essentially pure recombinant IL-1 protein and a pharmaceutically acceptable excipient.

21. A composition according to any one of the preceding claims, for use in inducing the immune system to respond after being suppressed as a result of chemotherapy, radiotherapy or disease.

13

EP 0 391 444 A2

TIME: 0 (INITIAL)

SDS PAGE                    INITIAL

| LANE | TITLE | MW |
|------|-------|-----|
| 1 | CM | - |
| 2 | IL-1 | 17742 |
| 3 | IL-1 | 17742 |
| 4 | IL-1 | 17742 |
| 5 | CM | - |
| 6 | IL-1 | 17742 |
| 7 | IL-1 | 17742 |
| 8 | IL-1 | 17742 |

CM = CALIBRATION MARKER

| | |
|--|--|
| 1 | 74,000 |
| 2 | 67,000 |
| 3 | 43,000 |
| 4 | 30,000 |
| 5 | 20,100 |
| 6 | 14,400 |

*FIG._1A.*

TIME: 12 MONTHS

12.0 MO

| LANE | TITLE | MW |
|------|-------|-------|
| 1 | CM | - |
| 2 | 5 | 17386 |
| 3 | RT | 17186 |
| 4 | 30 | 17186 |
| 5 | 40 | 16695 |
| 6 | CM | - |

FIG._1B.